# EUROPEAN PATENT APPLICATION

(11) **EP 2 206 726 A1**
(43) Date of publication of application: **14.07.2010**
(21) Application number: 09305016.9
(22) Date of filing: 08.01.2009
(51) Int. Cl.: C07K 14/705, A61K 38/17, C07K 7/06, A61K 38/08, A61K 49/00, A61P 9/10

(54) **Non-invasive tools for detecting vulnerable atherosclerotic plaques**

(71) Applicant: UNIVERSITE JOSEPH FOURIER, F-38041 Grenoble Cédex 9 (FR)
(72) Inventor: Fagret, Daniel, 38000, GRENOBLE (FR); Ghezzi, Catherine, 38000, GRENOBLE (FR); Dumy, Pascal, 38000, GRENOBLE (FR); Riou, Laurent, 38100, GRENOBLE (FR); Boturyn, Didier, 38500, LA BUISSE (FR)
(74) Representative: Jacobson, Claude

(57) **Abstract**

The present invention relates to a polypeptide comprising or constituted of the sequence RILAR (SEQ ID NO: 3), the use thereof in imaging methods, in diagnostic or prognostic methods and for use as a medicament.

SEQUENCE LISTING

<110>  Université Joseph Fourier

<120>  Non-invasive tools for detecting vulnerable atherosclerotic
       plaques

<130>  BET 08P0966

<160>  29

<170>  PatentIn version 3.4

<210>  1
<211>  10
<212>  PRT
<213>  Artificial

<220>
<223>  B2702-p

<400>  1

Arg Glu Asn Leu Arg Ile Ala Leu Arg Tyr
1               5                   10


<210>  2
<211>  20
<212>  PRT
<213>  Artificial

<220>
<223>  B2702-rp

<400>  2

Tyr Arg Leu Ala Ile Arg Leu Asn Glu Arg Arg Glu Asn Leu Arg Ile
1               5                   10                  15


Ala Leu Arg Tyr
            20


<210>  3
<211>  5
<212>  PRT
<213>  Artificial

<220>
<223>  peptide RILAR

<400>  3

Arg Ile Leu Ala Arg
1               5


<210>  4
<211>  6
<212>  PRT
<213>  Artificial

<220>
<223>  peptide RILARY

<400>  4

Arg Ile Leu Ala Arg Tyr
1               5

<210>  5
<211>  7
<212>  PRT
<213>  Artificial

<220>
<223>  peptide LRILARY

<400>  5

Leu Arg Ile Leu Ala Arg Tyr
1               5

<210>  6
<211>  7
<212>  PRT
<213>  Artificial

<220>
<223>  peptide NLRILAR

<400>  6

Asn Leu Arg Ile Leu Ala Arg
1               5

<210>  7
<211>  9
<212>  PRT
<213>  Artificial

<220>
<223>  peptide ANRILARY

<400>  7

Ala Asn Leu Arg Ile Leu Ala Arg Tyr
1               5

<210>  8
<211>  10
<212>  PRT
<213>  Artificial

<220>
<223>  EP43-HG

<400>  8

Arg Ala Asn Leu Arg Ile Leu Ala Arg Tyr
1               5                   10

<210>  9
<211>  12
<212>  PRT
<213>  Artificial

<220>
<223>  EP43

<400>  9

His Gly Arg Ala Asn Leu Arg Ile Leu Ala Arg Tyr
1               5                   10


<210>  10
<211>  12
<212>  PRT
<213>  Artificial

<220>
<223>  EP35

<400>  10

His Gly Arg Glu Asn Leu Arg Ile Leu Ala Arg Tyr
1               5                   10


<210>  11
<211>  12
<212>  PRT
<213>  Artificial

<220>
<223>  EP36

<400>  11

His Gly Arg Glu Asn Leu Arg Ile Leu Ala Arg Ala
1               5                   10


<210>  12
<211>  12
<212>  PRT
<213>  Artificial

<220>
<223>  EP37

<400>  12

His Gly Arg Glu Asn Leu Arg Ile Leu Ala Ala Tyr
1               5                   10


<210>  13
<211>  12
<212>  PRT
<213>  Artificial

<220>
<223>  EP38

<400>  13

His Gly Arg Glu Asn Leu Arg Ile Ala Ala Arg Tyr
1               5                   10


<210>  14
<211>  12
<212>  PRT
<213>  Artificial

<220>
<223>  EP40

<400>  14

His Gly Arg Glu Asn Leu Ala Ile Leu Ala Arg Tyr
1               5                   10


<210>  15
<211>  12
<212>  PRT
<213>  Artificial

<220>
<223>  EP41

<400>  15

His Gly Arg Glu Asn Ala Arg Ile Leu Ala Arg Tyr
1               5                   10


<210>  16
<211>  12
<212>  PRT
<213>  Artificial

<220>
<223>  EP42

<400>  16

His Gly Arg Glu Ala Leu Arg Ile Leu Ala Arg Tyr
1               5                   10


<210>  17
<211>  12
<212>  PRT
<213>  Artificial

<220>
<223>  EP44

<400>  17

His Gly Ala Glu Asn Leu Arg Ile Leu Ala Arg Tyr
1               5                   10


<210>  18
<211>  12
<212>  PRT
<213>  Artificial

<220>
<223>  EP51

<400>  18

His Gly Arg Ala Asn Leu Arg Ile Leu Ala Arg Ala
1               5                   10


<210>  19
<211>  12
<212>  PRT
<213>  Artificial

<220>
<223>  EP52

<400>  19

His Gly Arg Ala Asn Leu Arg Ile Leu Ala Ala Tyr
1               5                   10


<210>  20
<211>  10
<212>  PRT
<213>  Artificial

<220>
<223>  EP35-HG

<400>  20

Arg Glu Asn Leu Arg Ile Leu Ala Arg Tyr
1               5                   10


<210>  21
<211>  10
<212>  PRT
<213>  Artificial

<220>
<223>  EP36-HG

<400>  21

Arg Glu Asn Leu Arg Ile Leu Ala Arg Ala
1               5                   10


<210>  22
<211>  10
<212>  PRT
<213>  Artificial

<220>
<223>  EP37-HG

<400>  22

Arg Glu Asn Leu Arg Ile Leu Ala Ala Tyr
1               5                   10


<210>  23
<211>  10
<212>  PRT
<213>  Artificial

<220>
<223>  EP38-HG

<400>  23

Arg Glu Asn Leu Arg Ile Ala Ala Arg Tyr
1               5                   10


<210>  24
<211>  10
<212>  PRT
<213>  Artificial

<220>

<223>  EP40-HG

<400>  24

Arg Glu Asn Leu Ala Ile Leu Ala Arg Tyr
1               5                   10


<210>  25
<211>  10
<212>  PRT
<213>  Artificial

<220>
<223>  EP41-HG

<400>  25

Arg Glu Asn Ala Arg Ile Leu Ala Arg Tyr
1               5                   10


<210>  26
<211>  10
<212>  PRT
<213>  Artificial

<220>
<223>  EP42-HG

<400>  26

Arg Glu Ala Leu Arg Ile Leu Ala Arg Tyr
1               5                   10


<210>  27
<211>  10
<212>  PRT
<213>  Artificial

<220>
<223>  EP44-HG

<400>  27

Ala Glu Asn Leu Arg Ile Leu Ala Arg Tyr
1               5                   10


<210>  28
<211>  10
<212>  PRT
<213>  Artificial

<220>
<223>  EP51-HG

<400>  28

Arg Ala Asn Leu Arg Ile Leu Ala Arg Ala
1               5                   10


<210>  29
<211>  10
<212>  PRT
<213>  Artificial

<220>
<223>  EP52-HG

<400>  29

Arg Ala Asn Leu Arg Ile Leu Ala Ala Tyr
1               5                   10

## Description

The present invention relates to non-invasive tools for detecting vulnerable atherosclerotic plaques.

Cardiovascular diseases, and in particular coronary events, are the leading cause of mortality worldwide. Coronary events are mainly caused by the rupture or erosion of a vulnerable coronary atherosclerotic plaque and subsequent thrombus formation. However, no effective non-invasive tool is available for the detection of vulnerable plaques, which would enable efficient prevention of coronary events.

Vulnerable plaques are characterized by a thin fibrous cap surrounding a large lipidic and necrotic core and by intense ongoing inflammation. The inflammatory process leading to the development of vulnerable atherosclerotic lesions is characterized by extensive recruitment of monocytes and lymphocytes into the arterial wall.

Several endothelial adhesion molecules are implicated in the process of leucocyte rolling, firm adhesion and transmigration, such as VCAM-1. VCAM-1 is an endothelial adhesion molecule involved in the adhesion of monocytes to the vascular endothelium which has been shown to be overexpressed at the surface of vulnerable coronary atherosclerotic plaques.

Peptides consisting of residues 75-84 and 84-75/75-84 of the major histocompatibility complex-1 (MHC-1) molecule B2702, corresponding respectively to B2702-p, RENLRIALRY (SEQ ID NO: 1) and B2702-rp, YRLAIRLNER RENLRIALRY (SEQ ID NO: 2) bind specifically to VCAM-1 (Ling et al. (2000) Transplantation 70:662-667). It has been shown that these peptides could be used as specific markers of the vulnerable coronary atheroma plaque, in particular, in non-invasive imaging methods. The results indeed indicated that B2702-p activity colocalized with areas of plaque development expressing VCAM-1 on *ex vivo* autoradiographic images of aortas, with significantly higher tracer activity in zones of lipid accumulation than in normal areas of the arteries (Broisat et al. (2007) Eur. J. Nucl. Med. Mol. Imaging 34:830-840). However, *in vivo* imaging was not optimal due to high basal levels of circulating B2702-p.

The present invention arises from the unexpected finding by the inventors that mutated derivatives of B2702-p displayed improved characteristics for the *in vivo* imaging of VCAM-1 expression.

### Summary of the invention

The present invention thus relates to a polypeptide comprising or constituted of the sequence RILAR (SEQ ID NO: 3).

The present invention also relates to the polypeptide as defined above for use in imaging methods, in diagnostic or prognostic methods, or as a medicament.

The present invention also relates to the polypeptide as defined above for binding to VCAM-1.

The present invention also relates to an imaging method comprising the steps of:
a) administering a polypeptide as defined above to an individual;
b) detecting the binding or the absence of binding of the polypeptide in body areas of the individual;
c) visualizing the body areas of the individual wherein binding of the polypeptide can be detected.

The present invention also relates to a method of diagnosis and/or prognosis of a disease in an individual comprising the steps of:
a) administering a polypeptide as defined above to an individual,
b) detecting the binding or the absence of binding of the polypeptide in body areas of the individual;
c) visualizing the body areas of the individual wherein binding of the polypeptide can be detected,
d) deducing therefrom whether the individual suffers or will suffer of said disease.

The present invention also relates to the use of a polypeptide as defined above, for the *in vitro* detection of VCAM-1 in a sample.

### Detailed description of the invention

### Polypeptides

In the context of the invention, the terms "polypeptide" and "peptide" are used indifferently and refer to native peptides (either proteolysis products or synthetically synthesized peptides) and further to peptidomimetics, such as peptoids and semipeptoids which are peptide analogs, which may have, for example, modifications rendering the peptides more stable while in a body, or more immunogenic. Such modifications include, but are not limited to, cyclization, N-terminus modification, C-terminus modification, peptide bond modification, including, but not limited to, CH₂-NH, CH₂-S, CH₂-S=O, O=C-NH, CH₂-O, CH₂-CH₂, S=C-NH, CH=CH or CF=CH, backbone modification and residue modification. Methods for preparing peptidomimetic compounds are well known in the art and are specified in Quantitative Drug Design, CA. Ramsden Gd., Chapter 17.2, F. Choplin Pergamon Press (1992).

According to the invention, a peptide consist of less than 50 amino acids, preferably less than 40 amino acids, more preferably less than 30 amino acids, still preferably less than 20 amino acids. More preferably, peptides according to the invention have a length of from about 5 to about 18 amino acids, from about 5 to about 17 amino acids, from about 5 to about 16 amino acids, from about 5 to about 15 amino acids, from about 5 to about 14 amino acids, from about 5 to about 13 amino acids. Most preferably, peptides according to the invention have a length of 12, 11, 10, 9 or 8 amino acids.

As used herein, the term "amino acid" is understood to include: the 20 naturally occurring amino acids *i*.*e*. alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine; amino acids harbouring the post-translational modifications which can be found *in vivo* such as hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

As intended herein, the peptides of the invention can be substituted by one or more atoms or groups.

Preferably, the polypeptide according to the invention comprises or is constituted of the sequence RILARY (SEQ ID NO: 4). More preferably, the polypeptide according to the invention comprises or is constituted of the sequence LRILARY (SEQ ID NO: 5). Still preferably, the polypeptide according to the invention comprises or is constituted of the sequence NLRILAR (SEQ ID NO: 6). More preferably, the polypeptide according to the invention comprises or is constituted of the sequence ANLRILARY (SEQ ID NO: 7). More preferably, the polypeptide according to the invention comprises or is constituted of the sequence RANLRILARY (SEQ ID NO: 8). Most preferably, the polypeptide according to the invention is constituted of the sequence HGRANLRILARY (called EP43, SEQ ID NO: 9).

The invention also relates to a polypeptide comprising or consisting of the sequence selected from the group consisting of HGRENLRILARY (called EP35, SEQ ID NO: 10), HGRENLRILARA (called EP36, SEQ ID NO: 11), HGRENLRILAAY (called EP37, SEQ ID NO: 12), HGRENLRIAARY (called EP 38, SEQ ID NO: 13), HGRENLAILARY (called EP40, SEQ ID NO: 14), HGRENARILARY (called EP41, SEQ ID NO: 15), HGREALRILARY (called EP42, SEQ ID NO: 16), HGAENLRILARY (called EP44, SEQ ID NO: 17), HGRANLRILARA (called EP51, SEQ ID NO: 18), HGRANLRILAAY (called EP52, SEQ ID NO: 19), RENLRILARY (SEQ ID NO: 20), RENLRILARA (SEQ ID NO: 21), RENLRILAAY (SEQ ID NO: 22), RENLRIAARY (SEQ ID NO: 23), RENLAILARY (SEQ ID NO: 24), RENARILARY (SEQ ID NO: 25), REALRILARY (SEQ ID NO: 26), AENLRILARY (SEQ ID NO: 27), RANLRILARA (SEQ ID NO: 28) and RANLRILAAY (SEQ ID NO: 29).

### Detectable markers

In a particular embodiment, the polypeptide as defined above may be substituted with a detectable marker.

As used herein, a "detectable marker" refers to a compound which produces a signal that may be detected. When it is associated with a molecule, such as the polypeptide as defined above, it allows monitoring the fate of the molecule in the organism. Examples of detectable markers include radioisotopes, fluorophores such as Fluoresceine, Alexa, Cyanine, chemoluminescent compounds such as luminol, bioluminescent compounds such as luciferase or alkaline phosphatase, and contrast agents such as nanoparticules or Gadolinium. The choice of the suitable detectable marker, which depends on the detection system used, is well within the scope of the person skilled in the art.

Preferably, the detectable marker is a radioisotope. Examples of radioisotopes more particularly used in nuclear imaging include iodine 123 (¹²³I), iodine 125 (¹²⁵I), technetium 99m (^{99m}Tc), indium 111 (¹¹¹In), fluorine 18 (¹⁸F), gallium 67 (⁶⁷Ga), gallium 68 (⁶⁸Ga) and any other radioisotope usable in human beings. Accordingly, the radioisotope is preferably selected from the group constituted of ¹²³I, ¹²⁵I, ^{99m}Tc, ¹¹¹In, ¹⁸F, ⁶⁷Ga, or ⁶⁸Ga.

As used herein, the expression "polypeptide substituted with a detectable marker" means that a moiety of an amino acid residue of said polypeptide is substituted with a detectable marker. For example, an hydrogen atom may be substituted by an iodine atom in tyrosine residues.

In a preferred embodiment; the polypeptide according to the invention is constituted of HGRANLRILARY-¹²³I or ^{99m}Tc-HGRANLRILARY.

### Imaging methods

In the context of the invention, "imaging methods" refer to methods that enable visualizing the inside of an organism or organs of an organism. Examples of imaging methods encompass invasive techniques such as angiography or coronarography, endocoronarian ecography, and non-invasive techniques such as Doppler velocimetry, angiography by magnetic resonance imaging or nuclear medicine such as scintigraphy. Preferably, the imaging method according to the invention is scintigraphy.

In a particular embodiment, the polypeptide as defined above is used in imaging methods for detecting atherosclerotic plaques.

In the context of the invention, an "atherosclerotic plaque" or "atheroma plaque" refers to a lesion of vessel walls. Preferably, an "atherosclerotic plaque" according to the invention comprises a lipid core and a fibrous cap, said cap being constituted by smooth muscle cells, collagens and an extracellular matrix and isolating the lipid core from the arterial lumen. Atherosclerotic plaques may be found for example in the aorta, the carotid, or in the coronary artery. When the plaque comprises a thin fibrous cap (about 65 to 150 µm thick) and a considerable lipid core, it is referred to as "vulnerable atherosclerotic plaque" or "vulnerable atheroma plaque" or "vulnerable plaque". These instable plaques, which are prone to rupture, may be found in coronary arteries and in aorta and its branches.

Preferably, the polypeptide as defined above is used in imaging methods for detecting vulnerable atherosclerotic plaques. More preferably, the polypeptide as defined above is used in imaging methods for detecting aortic, carotidian, or coronary atherosclerotic plaques.

The present invention also relates to an imaging method comprising the steps of:
a) administering a polypeptide as defined above to an individual;
b) detecting the binding or the absence of binding of the polypeptide in body areas of the individual;
c) visualizing the body areas of the individuals wherein binding of the polypeptide can be detected.

In the context of the present invention, an "individual" denotes a human or non-human mammal, such as a rodent (rat, mouse, rabbit), a primate (chimpanzee), a feline (cat), a canine (dog). Preferably, the individual is human.

Any suitable method of administration, known from one skilled in the art may be used in step a). In particular, the polypeptide may be administered for example by the oral route, by inhalation, or by the parenteral route (in particular by intravenous injection). When the parenteral route is selected, the polypeptide may be in the form of injectable solutions and suspensions, conditioned in ampoules or flasks. The forms for parenteral delivery are conventionally obtained by mixing the polypeptide according to the invention with buffers, stabilizers, preservatives, solubilizing agents, isotonic agents and slurrying agents. According to known techniques, these mixtures can then be sterilized and conditioned in the form of intravenous injections. One of skill in the art may use organic phosphate salts-based buffers as buffer. Examples of slurrying agents include methylcellulose, acacia and sodium carboxymethylcellulose. Examples of stabilizers include sodium sulphite and sodium metasulphite, and examples of preservatives include sodium p-hydroxybenzoate, sorbic acid, cresol and chlorocresol.

The amount of polypeptide administered naturally depends on the administration route, the size and/or weight of the individual, and the detection technique used.

As used herein, the expression "body area" refers to a determined region of the organism. It may be for example an organ, a part of an organ, or a tissue, such as a lung, the heart, the liver, the spleen, or a kidney, or a blood vessel such as an artery or a vein.

Any suitable system of detection and visualization, known from the one skilled in the art may be used in steps b) and c) defined above. In particular, steps b) and c) may be carried by scintigraphy. In this case, the polypeptide used is preferably substituted with a radioisotope selected from the group constituted of ¹²³I, ¹²⁵I, ^{99m}Tc, ¹¹¹In, ¹⁸F, ⁶⁷Ga or ⁶⁸Ga. More preferably, the polypeptide used is constituted of HGRANLRILARY-¹²³I or ^{99m}Tc-HGRANLRILARY.

In a preferred embodiment, the imaging method as defined above is used for visualizing atherosclerotic plaques of the individual. In particular, the imaging method as defined above may be used for visualizing aortic, carotidian, or coronary atherosclerotic plaques of the individual.

### Diagnostic and prognostic methods

As used herein, a "diagnostic method" or "diagnosis" refers to a method for determining whether an individual suffers from a pathology.

As used herein, a "prognostic method" or "prognosis" refers to a method for determining whether an individual is likely to develop a pathology.

Preferably, the polypeptide as defined above is for use in the diagnosis of cardiovascular diseases.

In the context of the invention, a "cardiovascular disease" refers to a disease that involves the heart or blood vessels (arteries and veins). More particularly, a cardiovascular disease according to the invention denotes a disease, lesion or symptom associated with an atherogenesis process that affects the cardiovascular system. It includes especially the conditions in which an atheroma plaque develops as well as the complications due to the formation of an atheroma plaque (stenosis, ischemia) and/or due to its evolution toward an acute ischemic stroke (thrombosis, embolism, infarction, arterial rupture). Cardiovascular diseases include atherosclerosis, atheroma plaque, in particular vulnerable plaque, coronary heart disease, angina pectoris, thrombosis, stroke, myocardial infarction, vascular stenosis, and infarction. More preferably, the polypeptide as defined above is for use for diagnosing atherosclerosis or a coronary heart disease.

As used herein, "atherosclerosis" denotes a disease affecting arterial blood vessels. Atherosclerosis can be characterized by a chronic inflammatory response in the walls of arteries, mainly due to the accumulation of macrophages and promoted by low density lipoproteins without adequate removal of fats and cholesterol from macrophages by functional high density lipoproteins.

As used herein, a "coronary heart disease" denotes a progressive disease, due to a bad irrigation of the heart muscle, consecutive to the narrowing (stenosis) or calcification (sclerosis) of a coronary artery. The complete obstruction of a coronary artery leads to myocardial infarction.

In a particular embodiment, the polypeptide as defined above is for use for assessing the risk of occurrence of an acute ischemic stroke.

As used herein, the expression "risk of occurrence" refers to the probability that a subject develop a pathology.

As used herein, an "acute ischemic stroke" denotes the decrease of arterial blood uptake to a region of the organism. Its main local causes are thrombosis and embolism. When the atheroma plaque is located in the coronary artery, the acute ischemic stroke is in particular a myocardial infarction. When it is located in a carotid artery, it may lead to cerebral vascular accident. When it is located in a renal artery, it may lead to a renal artery embolism. When it is located in an artery of a limb, it may lead to an acute ischemic stroke of a limb.

Therefore, the polypeptide as defined above is preferably for use for assessing the risk of occurrence of an acute ischemic stroke selected from the group consisting of a myocardial infarction, a cerebral vascular accident, a renal artery embolism, and an acute ischemic stroke of a limb.

### Medicament

A method of treatment comprising the administration of a therapeutically effective amount of the polypeptide as defined above is also include in the present invention.

The polypeptide as defined above is advantageously coupled to a cytotoxic agent, so that the cytotoxic agent is contacted with a VCAM-1 expressing cell and selectively destroys this cell. The selection of a suitable cytotoxic agent is within the scope of one skilled in the art. Examples of cytotoxic agents include radioisotopes, toxins or chemotherapeutic agents.

The invention relates more particularly to the polypeptide as defined above for use for treating atherosclerosis and/or preventing an acute ischemic stroke, more preferably, for treating a vulnerable atherosclerotic plaque.

### Binding and detection of VCAM-1

The present invention also relates to the polypeptide as defined above for binding to VCAM-1.

"VCAM-1" (Vascular Cell Adhesion Molecule 1) denotes a cellular adhesion protein, the transcription of which is induced in endothelial cells but which is also expressed in other cell types. VCAM-1 was identified and cloned by Osborn *et al.* in 1989 (Osborn et al. (1989) Cell 6:1203-11). VCAM-1 interacts with integrin α₄β₁, also called VLA-4 (Very Late Antigen 4), which is expressed constitutively in lymphocytes and monocytes. Like other adhesion molecules, such as ICAM-1, 2 and 3, VCAM-1 is involved in the adhesion of monocytes to the endothelium during atherosclerosis. VCAM-1 also interacts with integrin α₄β₇ to recruit lymphocytes in the intestine.

The polypeptide as defined above may also be used for the *in vitro* detection of VCAM-1 in a sample.

As used herein, a "sample" refers to a part of a bigger set. Preferably, a sample according to the invention is a substance of biological origin. Examples of biological samples include, but are not limited to, pieces of organs or of tissues such as kidney, liver, heart, lung, and the like, arteries veins and the like, blood and components thereof such as plasma, platelets, subpopulations of blood cells and the like.

The following examples and figures illustrate the invention without limiting its scope.

### Brief description of the figures

**Figure 1** shows histograms representing the left-to-right carotid artery activity ratio obtained from gamma-well counting of excised vessels and blood from mice injected with radiolabelled peptides B2702-p, EP35, EP36, EP37, EP38, EP40, EP41, EP42, EP43 or EP44. * : p<0.05 vs B2702-p.
**Figure 2** shows histograms representing the left carotid-to-blood activity ratio obtained from gamma-well counting of excised vessels and blood from mice injected with radiolabelled peptides B2702-p, EP35, EP36, EP37, EP38, EP40, EP41, EP42, EP43 or EP44. * : p<0.05 vs B2702-p.
**Figure 3** shows histograms representing the tissue activity (in %ID/g) in different organs (heart, aorta, lung, liver, spleen, kidney, fat, skeletal muscle, blood, thyroid) after injection of radiolabelled B2702-p (white bars), EP43 (diagonal double hatched bars), EP36 (diagonal hatched bars), EP37 (vertical hatched bars), EP51 (horizontal hatched bars) and EP52 (last diagonal hatched bars).
**Figure 4** shows histograms representing the left-to-right carotid artery activity ratio obtained from mice injected with radiolabelled peptides B2702-p, EP43, E36, EP37, EP51 and EP52. * : p<0.05 vs B2702-p.
**Figure 5** shows histograms representing the left carotid-to-blood activity ratio obtained from mice injected with radiolabelled peptides B2702-p, EP43, E36, EP37, EP51 and EP52. * : p<0.05 vs B2702-p.
**Figure 6** shows representative whole-body planar images obtained with mice injected with radiolabelled B2702-p (left) or radiolabelled EP43 (right). The white arrow indicates the atherosclerotic lesion. The scale at the bottom indicates the color look-up table (LUT) used to display the image.
**Figure 7** shows histograms representing the quantifications of planar images obtained with mice injected with radiolabelled B2702-p, EP35, EP36, EP37, EP38, EP40, EP41, EP42, EP43 and EP44. * : p<0.05 vs B2702-p.
**Figure 8** shows representative whole-body planar images obtained with mice injected with radiolabelled B2702-p (first from the left), radiolabelled EP43 (second from the left), radiolabelled EP51 (third from the left) or radiolabelled EP52 (fourth from the left). The white arrow indicates the atherosclerotic lesion. R indicates the right side of the animal and L indicates the left side of the animal. The scale at the bottom indicates the color look-up table (LUT) used to display the image.
**Figure 9** shows histograms representing the quantifications of planar images obtained with mice injected with radiolabelled B2702-p, EP43, EP36, EP37, EP51 and EP52. * : p<0.05 vs B2702-p.
**Figure 10** shows a representative transversal image of EP43 activity, obtained by high resolution pinhole SPECT imaging. The white arrow indicates EP43 uptake at the level of the atherosclerotic lesion. The scale at the bottom indicates the color look-up table used to display the image.
**Figure 11** shows a graph representing quantification (expressed in cpm/mm²/MBq) of images, obtained by high resolution pinhole SPECT imaging performed on mice injected with EP43, corresponding to the activity in the left carotid and in the right carotid. White circles indicate the results obtained for each animal tested. Black circles indicate the mean value. *: p<0.01 vs right carotid.

### Example

The objective of this study was to determine whether mutated derivatives of B2702-p (SEQ ID NO: 1) displayed improved characteristics for the *in vivo* imaging of VCAM-1 expression.

### Material and methods

### Polypeptides and tracers

Eleven derivatives of B2702-p were obtained by site-directed mutagenesis by selectively substituting each residue of the B2702-p sequence by an alanine residue.

The sequences of the eleven derivatives are listed in **Table 1.**

**Table 1: Sequence of the B2702-p derivatives**

| **Name** | **Peptidic sequence** | **SEQ ID NO** |
|---|---|---|
| B2702-p | HGRENLRIALRY | 1 |
| EP35 | HGRENLRILARY | 10 |
| EP36 | HGRENLRILARA | 11 |
| EP37 | HGRENLRILAAY | 12 |
| EP38 | HGRENLRIAARY | 13 |
| EP40 | HGRENLAILARY | 14 |
| EP41 | HGRENARILARY | 15 |
| EP42 | HGREALRILARY | 16 |
| EP43 | HGRANLRILARY | 9 |
| EP44 | HGAENLRILARY | 17 |
| EP51 | HGRANLRILARA | 18 |
| EP52 | HGRANLRILAAY | 19 |

The polypeptides were labelled with ^{99m}Tc as described in Broisat et al. (2007) Eur. J. Nucl. Med. Mol. Imaging 34:830-840.

### Animal model and experimental protocol

An experimental model of ApoE^{-/-} mice with left carotid artery ligation was used. Indeed, hypercholesterolemia associated with the vascular remodelling induced by carotid artery ligation induces the development of a focal atherosclerotic lesion at the site of ligation.

Left carotid artery ligation was performed under anesthesia and the animals were returned to their cages. Three weeks later, the animals were re-anesthetized and the radiotracers were injected in a lateral tail vein. Planar imaging was performed for 3 hours before the animals were euthanized. The major organs were excised, weighed and gamma-well counted for the determination of the tracers' biodistribution. Immunohistochemistry was also performed to evaluate VCAM-1 expression in the atherosclerotic lesion.

The experimental model was validated by assessing the presence of VCAM-1 in the atherosclerotic lesion using immunohistochemistry. The inventors observed a positive VCAM-1 staining in the atherosclerotic lesion as compared with a contralateral vessel.

### In vivo planar imaging

*In vivo* planar imaging was performed using a small-animal dedicated gamma-camera equipped with a parallel-hole collimator.

### In vivo pinhole SPECT imaging

*In vivo* pinhole SPECT imaging was performed using a small-animal dedicated gamma-camera equipped with a pinhole collimator for high-resolution imaging.

### Results

### Organ biodistribution

Organ biodistributions of B2702-p and polypeptides EP35, EP36, EP37, EP38, EP40, EP41, EP42, EP43 and EP44 3 hours after injection are shown in **Table 2.**

**Table 2: Organ distribution of ^{99m}Tc-B2702-p and derivatives at 180 min following tracer injection**

| **Organs** | **Tracers** | | | | | | | | | |
|---|---|---|---|---|---|---|---|---|---|---|
| | **B2702** | **EP35** | **EP36** | **EP37** | **EP38** | **EP40** | **EP41** | **EP42** | **EP43** | **EP44** |
| **Heart** | 1.3 ± 0.1 | 0.5 ± 0.3 | 0.4 ± 0.1 | 0.3 ± 0.1 | 0.3 ± 0.1 | 0.3 ± 0.1 | 0.3 ± 0.0 | 0.1 ± 0.0 | 0.3 ± 0.1 | 0.4 ± 0.0 |
| **Aorta** | 2.1 ± 0.2 | 0.3 ± 0.0 | 0.7 ± 0.2 | 0.6 ± 0.3 | 0.7 ± 0.1 | 0.9 ± 0.6 | 0.6 ± 0.0 | 0.3 ± 0.0 | 0.5 ± 0.1 | 0.7 ± 0.1 |
| **Lung** | 3.8 ± 0.5 | 0.4 ± 0.1 | 0.9 ± 0.2 | 0.6 ± 0.2 | 0.9 ± 0.1 | 0.8 ± 0.3 | 0.7 ± 0.0 | 0.4 ± 0.0 | 0.8 ± 0.1 | 0.9 ± 0.1 |
| **Liver** | 12.7 ± 3.3 | 2.8 ± 0.5 | 4.2 ± 0.6 | 4.7 ± 0.9 | 6.9 ± 1.6 | 2.7 ± 0.3 | 5. 9 ± 1.0 | 2.1 ± 0.1 | 3.1 ± 0.6 | 8.2 ± 0.4 |
| **Spleen** | 2.3 ± 0.3 | 0.4 ± 0.1 | 0.8 ± 0.2 | 0.6 ± 0.2 | 0.8 ± 0.2 | 1.4 ± 0.4 | 0.5 ± 0.0 | 0.4 ± 0.1 | 1.6 ± 0.7 | 1.0 ± 0.3 |
| **Kidney** | 62.4 ± 17.3 | 23.6 ± 3.7 | 33.1 ± 0.7 | 26.2 ± 5.1 | 20.6 ± 10.8 | 13.4 ± 1.9 | 8.2 ± 0.7 | 2.2 ± 0.2 | 4.6 ± 0.5 | 8.0 ± 1.3 |
| **Fat** | 0.7 ± 0.1 | 0.1 ± 0.0 | 0.3 ± 0.1 | 0.2 ± 0.1 | 0.2 ± 0.1 | 0.3 ± 0.2 | 0.2 ± 0.0 | 0.1 ± 0.0 | 0.2 ± 0.0 | 0.2 ± 0.0 |
| **Skeletal muscle** | 1.2 ± 0.3 | 0.1 ± 0.0 | 0.2 ± 0.1 | 0.2 ± 0.1 | 0.2 ± 0.1 | 0.2 ± 0.1 | 0.1 ± 0.0 | 0.1 ± 0.0 | 0.1 ± 0.0 | 0.2 ± 0.0 |
| **Blood** | 5.2 ± 0.5 | 0.4 ± 0.0 | 1.3 ± 0.3 | 0.9 ± 0.4 | 1.2 ± 0.3 | 0.9 ± 0.3 | 1.1 ± 0.1 | 0.4 ± 0.0 | 0.9 ± 0.2 | 1.3 ± 0.1 |
| **Carotid artery,** | | | | | | | | | | |
| **Left** | 2.1 ± 0.5 | 0.1 ± 0.0 | 0.5 ± 0.1 | 0.7 ± 0.4 | 0.4 ± 0.1 | 0.5 ± 0.2 | 0.5 ± 0.1 | 0.1 ± 0.0 | 1.3 ± 0.4* | 0.4 ± 0.2 |
| **Right** | 1.6 ± 0.3 | 0.4 ± 0.2 | 0.4 ± 0.1 | 0.2 ± 0.1 | 0.2 ± 0.1 | 0.3 ± 0.1 | 0.4 ± 0.2 | 0.1 ± 0.0 | 0.5 ± 0.1 | 0.4 ± 0.2 |

The inventors observed that the elimination of the tracers was mainly renal and to a lower extent hepatic. The highest left carotid activity was observed following injection of EP43. The activity of the tracer in the atherosclerotic lesion was significantly higher than that observed in the contralateral, control carotid.

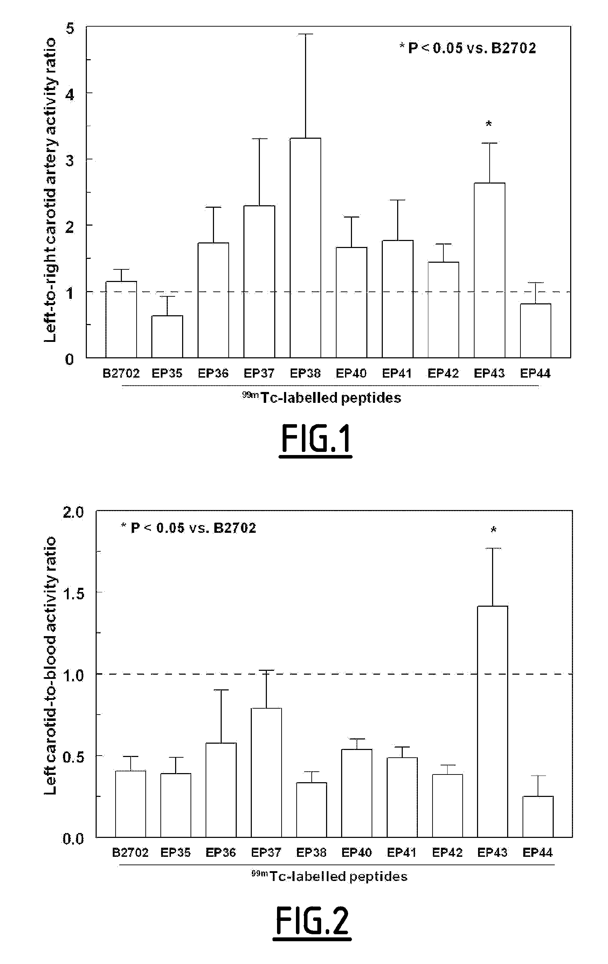

The inventors also analyzed the left-to-right carotid activity ratios **(****Figure 1****)** and left carotid-to-blood activity ratios **(****Figure 2****).** These ratios were obtained from gamma-well counting of excised vessels and blood.

The inventors demonstrated that EP43 left-to-right carotid activity and left carotid-to-blood activity ratios were both significantly higher than those of B2702-p. EP43 was the only derivative tested that displayed such improved characteristics with respect to B2702-p.

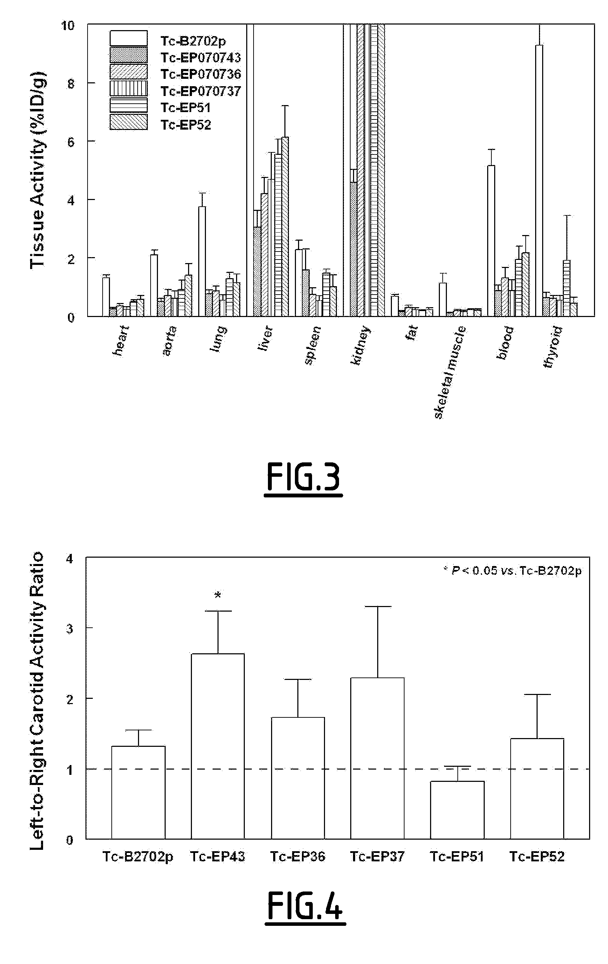

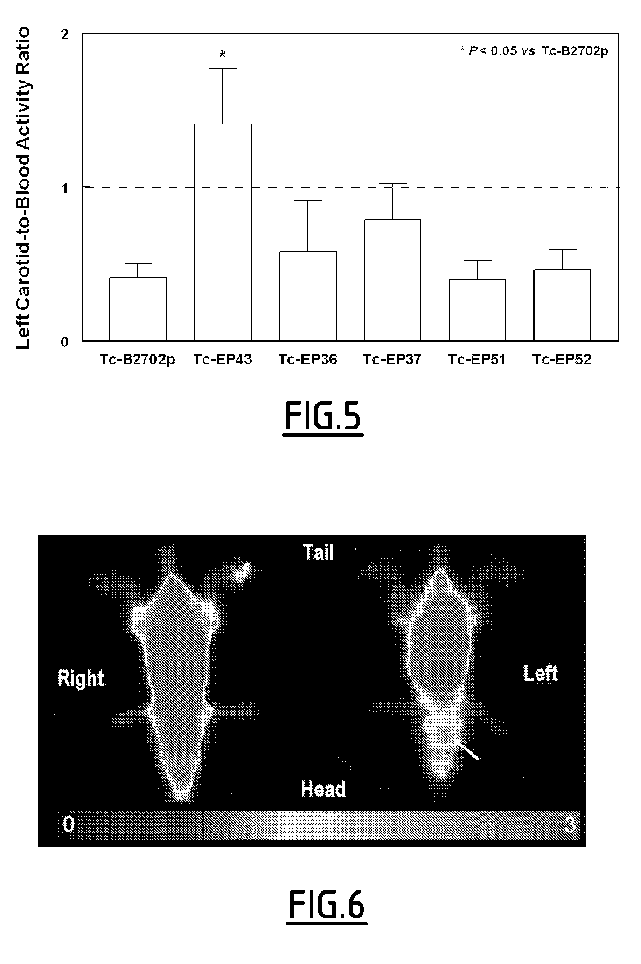

The inventors then analyzed the organ biodistributions **(****Figure 3****)** as well as the left-to-right carotid activity ratios **(****Figure 4****)** and left carotid-to-blood activity ratios **(****Figure 5****)** of B2702-p and polypeptides EP36, EP37, EP43, EP51 and EP52. They observed that EP51 and EP52 did not display improved left-to-right carotid artery activity ratios nor left carotid-to-blood activity ratios when compared with EP43.

### In vivo planar imaging

The inventors then performed *in vivo* whole-body planar imaging of mice injected with radiolabelled B2702-p and EP43 **(****Figure 6****).**

Images were obtained following injection of a similar dose of tracer. The high circulating blood activity precluded the non invasive imaging of B2702-p uptake in the carotid atherosclerotic lesion, whereas the improved characteristics of EP43 allowed the detection of VCAM-1 expression in the atherosclerotic lesion.

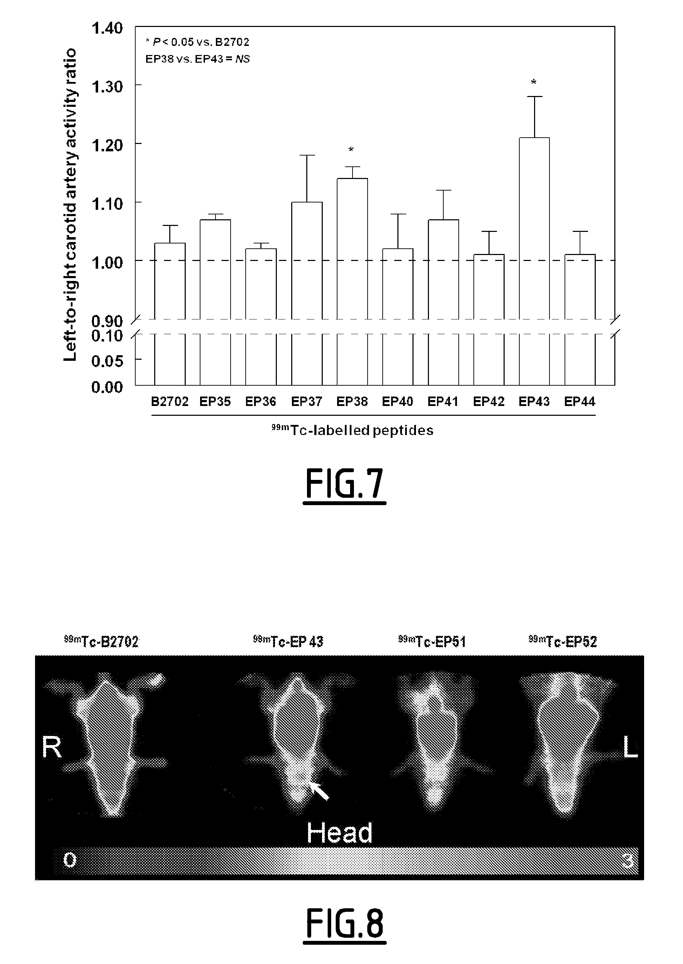

The inventors further quantified the above planar images **(****Figure 7****).** The results confirmed the superiority of EP43 over the other evaluated tracers for the non invasive detection of atherosclerosis.

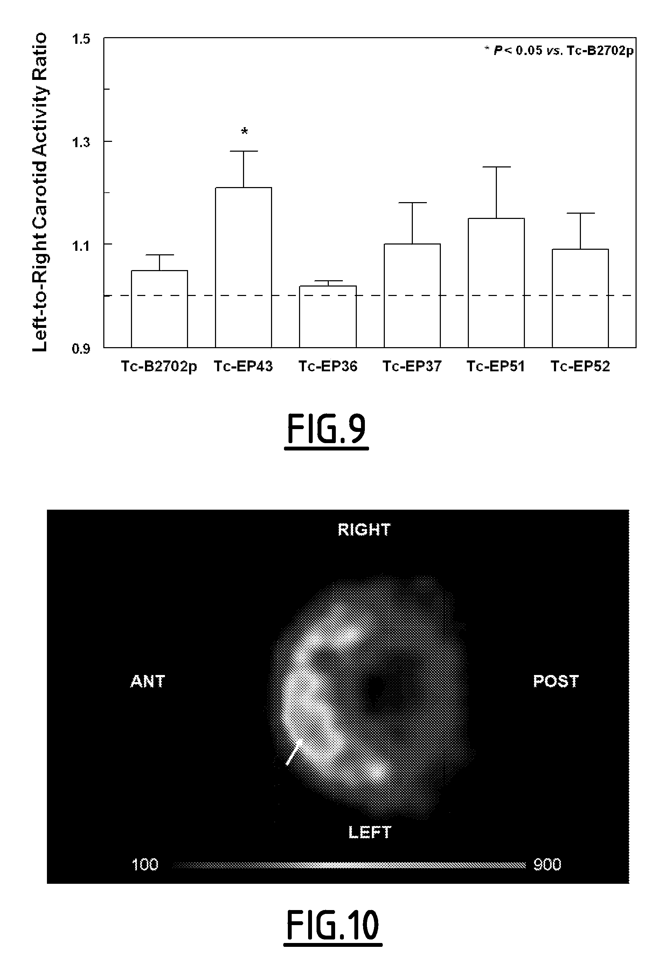

The inventors also performed *in vivo* whole-body planar imaging of mice injected with radiolabelled EP51 and EP52 **(****Figure 8****)** and quantified the planar images obtained **(****Figure 9****).** They observed that EP51 and EP52 did not display improved left-to-right carotid activity ratios when compared to EP43.

### In vivo pinhole SPECT imaging

High resolution pinhole SPECT imaging was also performed by the inventors following injection of EP43 to further confirm these results **(****Figure 10****).**

The inventors observed EP43 uptake at the level of the atherosclerotic lesion. In the other hand, no significant tracer activity could be detected in the right carotid area.

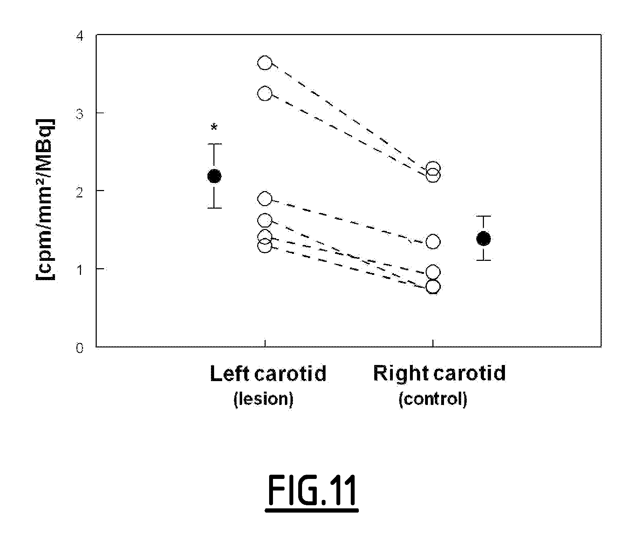

Image quantification was performed for all animals that underwent SPECT imaging of EP43 uptake **(****Figure 11****).** In all animals, there was higher tracer activity in the atherosclerotic lesion than in the contralateral vessel, resulting in a significantly higher mean tracer activity.

Accordingly, the present inventors have shown that the new radiolabelled polypeptide EP53 allowed *in vivo* SPECT imaging of VCAM-1 expression in atherosclerotic lesions, indicating that EP43 is a tracer useful for non-invasive imaging of atherosclerotic lesions in patients.

## Claims

1. A polypeptide comprising or constituted of the sequence RILAR (SEQ ID NO: 3).

2. The polypeptide according to claim 1, comprising or constituted of the sequence RANLRILARY (SEQ ID NO: 8).

3. The polypeptide according to claim 1 or 2, substituted with a detectable marker.

4. The polypeptide according to claim 3, wherein the detectable marker is a radioisotope selected from the group constituted of ¹²³I, ¹²⁵I, ^{99m}Tc, ¹¹¹In, ¹⁸F, ⁶⁷Ga, or ⁶⁸Ga.

5. The polypeptide according to any of claims 1 to 4, constituted of HGRANLRILARY-¹²³I or ^{99m}Tc-HGRANLRILARY.

6. The polypeptide according to any of claims 1 to 5, for use in imaging methods.

7. The polypeptide according to claim 6, for detecting atherosclerotic plaques.

8. The polypeptide according to any of claims 1 to 7, for use in diagnostic or prognostic methods.

9. The polypeptide according to any of claims 1 to 8, for diagnosing atherosclerosis or a coronary heart disease.

10. The polypeptide according to any of claims 1 to 8, for assessing the risk of occurrence of an acute ischemic stroke.

11. The polypeptide according to claim 1 or 2, for use as a medicament.

12. The polypeptide according to any of claims 1 to 11, for binding to VCAM-1.

13. An imaging method comprising the steps of:
a) administering a polypeptide as defined in any of claims 1 to 5 to an individual;
b) detecting the binding or the absence of binding of the polypeptide in body areas of the individual;
c) visualizing the body areas of the individual wherein binding of the polypeptide can be detected.

14. The imaging method according to claim 13, for visualizing atherosclerotic plaques of the individual.

15. The use of a polypeptide as defined in any of claims 1 to 5, for the *in vitro* detection of VCAM-1 in a sample.
